# EUROPÄISCHE PATENTSCHRIFT

(11) **EP 1 009 414 B1**
(45) Veröffentlichungstag und Bekanntmachung des Hinweises auf die Patenterteilung: **02.11.2005**
(21) Anmeldenummer: 98948878.8
(22) Anmeldetag: 27.08.1998
(51) Int. Cl.: A61K 31/71, A61P 15/02

(54) **PHARMAZEUTISCHE ZUSAMMENSETZUNG ENTHALTEND CLINDAMYCIN UND CLOTRIMAZOL, ZUR BEHANDLUNG VAGINALER INFEKTIONEN**
NOVEL PHARMACEUTICAL COMPOSITION COMPRISING CLINDAMYCIN AND CLOTRIMAZOL FOR THE TREATMENT OF VAGINAL INFECTIONS
NOUVELLE COMPOSITION PHARMACEUTIQUE CONTENANT CLINDAMYCIN ET CLOTRIMAZOL POUR LE TRAITMENT D'INFECTIONS VAGINALES

(30) Priorität: 27.08.1997 DE 19737348
(43) Veröffentlichungstag der Anmeldung: 21.06.2000
(73) Patentinhaber: Vulpescu, Dan-Gabriel, 27580 Bremerhaven (DE)
(72) Erfinder: VULPESCU, Dan-Gabriel, D-27580 Bremerhaven (DE); FREUDENSPRUNG, Brigitte, Hexal AG, D-83607 Holzkirchen (DE)
(86) Internationale Anmeldenummer: PCT/EP1998/005454
(87) Internationale Veröffentlichungsnummer: WO 1999/009964

(56) Entgegenhaltungen:
- US-A- 4 318 853
- US-A- 5 160 737
- HAY: "Recurrent Bacterial Vaginosis" DERMATOLOGIC CLINICS, Bd. 16, Nr. 4, Oktober 1998, Seiten 769-773, XP002098563
- SOBEL: "Vaginal Infections in Adult women" THE MEDICAL CLINICS OF NORTH AMERICA, Bd. 74, Nr. 6, November 1990, Seiten 1573-1602, XP002098564

## Beschreibung

Die Erfindung betrifft eine neue pharmazeutische Zusammensetzung enthaltend eine Kombination aus Clindamycin und Clotrimazol zur vaginalen Anwendung bei bakteriellen Infektionen, Pilz- oder Mischinfektionen der Vagina.

Herkömmliche Therapien zur. Behandlung von bakteriellen vaginalen Infektionen, wie z.B. bakterielle Vaginose, die durch das Zusammenwirken von Gardnerella vaginalis und anaeroben Bakterien zustande kommen, sehen Behandlungen mit Chemotherapeutika mit Wirkung auf Anaerobier und Protozoen, wie z.B: Metronidazol oder Tinidazol oder aber die Behandlung mit einem Antibiotikum wie Amoxicillin oder Clindamycin vor. Die Behandlung erfolgt entweder oral oder durch lokale, vaginale Applikation.

Die orale Therapie mit einem dieser Wirkstoffe besitzt erhebliche Nachteile, wie z.B. das Auftreten von erheblichen Nebenwirkungen oder aber auch unerwünschte Wechselwirkungen mit anderen Arzneimitteln. Zudem steht Metronidazol im Verdacht, kanzerogenes Potential zu haben. Eine Lokaltherapie ist auf jeden Fall einer systemischen Therapie vorzuziehen, um eine systemische Belastung des Körpers zu vermeiden.

Bisherige lokale Therapien mit Metronidazol sahen Dosierungen von 500 bis 1000 mg Metronidazol als Einmalgabe vor oder bei einer 5-Tage Therapie 200-500 mg Metronidazol pro Tag. Wird der Wirkstoff in einer halbfesten Zubereitung, wie z.B. als Gel oder Creme verabreicht, sind Gaben von ca. 100 mg pro Tag üblich. Analoge Mengen werden mit dem Wirkstoff Clindamycin angewandt. Jedoch werden auch bei der Lokaltherapie die gleichen oder ähnliche Nebenwirkungen beschrieben wie bei der oralen Anwendung der Wirkstoffe.

Ein wesentlicher Nachteil der oben beschriebenen Therapien mit einem Antibiotikum oder einem Chemotherapeutikum ist, daß sowohl bei oraler als auch bei lokaler Therapie häufig eine sekundäre Infektion z. B. Vaginalcandidose oder eine Mischinfektion innerhalb des Folgemonats auftreten kann, wodurch eine erneute Behandlung mit einem weiteren Medikament erforderlich wird. Eine sehr genaue und aufwendige Diagnose ist dann notwendig, um festzustellen, welche Art einer Kolpitis vorliegt, damit medikamentös richtig behandelt werden kann.

Eine der wohl am häufigsten auftretende Folgeinfektion ist die Vaginalcandidose, eine Pilzinfektion, die eine Behandlung mit einem Antimykotikum, wie z.B. Clotrimazol erforderlich macht.

Clotrimazol ist ein Lokal-Antimykotikum mit einem breitem Wirkungsspektrum, das viele humanpathogene Pilze umfaßt. In der Gynäkologie wird Clotrimazol vorwiegend zur Behandlung von vulvovaginalen Infektionen durch Hefe- oder Sproßpilze als Monotherapie eingesetzt. Bei der lokalen Therapie mit Clotrimazol werden Dosierungen von 500 mg bei der Einmalapplikation vorgesehen. Mehrtägige Behandlungstherapien werden mit Dosierungen von ca. 100-200 mg Clotrimazol durchgeführt.
Es ist des weiteren bekannt, daß neben der antimykotischen Wirkung Clotrimazol eine geringe antibakterielle Wirkung besitzt.

Aufgabe der Erfindung ist es, eine pharmazeutische Zusammensetzung zur Behandlung vaginaler Erkrankungen bereit zu stellen, bei der die Nebenwirkungen reduziert sind und mit der die Behandlung einfacher und effizienter wird.

Dies konnte durch eine neue pharmazeutische Zusammensetzung, die als aktive Bestandteile die Wirkstoffe Clindamycin und Clotrimazol enthält, erreicht werden.

Dabei wurde überraschenderweise gefunden, daß geringere Dosierungen erforderlich sind, als bei der Behandlung mit den einzelnen Wirkstoffen. Die Kombination zeigt einen deutlichen synergistischen Effekt. Während nach dem Stand der Technik Clindamycin bei der Lokaltherapie in Dosismengen von 100-200 mg pro Tag zu verabreichen ist, um eine vollständige Ausheilung der Erkrankung zu erzielen, kann bei der neuen Kombination die Clindamycindosis auf 10-20 mg reduziert werden. Je nach Schwere der Erkrankung werden Dosismengen von 10-20 mg Clindamycin in Kombination mit 50-100 mg Clotrimazol verabreicht.

Vorzugsweise ist die neue pharmazeutische Zusammensetzung zur lokalen Verwendung bei der Behandlung von vaginalen Infektionen bestimmt.

Die pharmazeutische Zusammensetzung kann neben den aktiven Bestandteilen pharmazeutische annehmbare Träger oder Hilfsstoffe enthalten. Bevorzugte Darreichungsformen sind Vaginalsuppositoria, Vaginaltabletten, Vaginalovula, Vaginalringe oder halbfeste Vaginalzubereitungen wie Salbe, Creme oder Gel.

Unter üblichen Tablettenhilfsstoffen werden hier die nachstehenden verstanden:
Stärke, z.B. Maisstärke, Reisstärke, Kartoffelstärke, Weizenstärke, Milchzucker (Lactose), Glucose, Saccharose, mikrokristalline Cellulose, Siliciumdioxid kollodial, Magnesiumstearat, Stearinsäure, Talcum, Polyvinylpyrrolidon (linear und quervernetzt), Natriumchlorid, Polyethylenglycol, Hydroxypropylmethylcellulose, Hydroxypropylcellulose, Gelatine, Calciumphosphat, Cellulose, Mannit, Natriumcarboxymethylstärke, Natriumcarbonat, Natriumbicarbonat, Calciumcarbonat, Natriumcarboxymethylcellulose (linear und quervernetzt) und Magnesiumstearat.
Weitere Tablettenhilfsstoffe siehe "Die Tablette, Grundlagen und Praxis des Tablettierens, Granulierens und Dragierens" von W.A. Ritschel, S.85-144, sowie "Katalog pharmazeutischer Hilfsstoffe" verfaßt von einer Arbeitsgruppe der Firma Ciba-Geigy, Hoffmann-La Roche und Sandoz, Basel 1974.

Unter üblichen Hilfsstoffen für Cremes werden hier die nachstehenden verstanden:
Sorbitanmonostearat, Polysorbat 60, Cetylpalmitat, Paraffin, Cetylstearylalkohol, Benzylalkohol, Siliciumdioxid, Triacetin, Isopropylmonostearat, Polyethylenglycol, Glycerolmonostearat, Polyacrylsäure, Natriumhydroxid, Docusat-Natrium, Dimethicon, Triglyceride, Octyldecanol und Octyldodecanol.

Unter üblichen Hilfsstoffen für Ovula werden hier die nachstehenden verstanden:
Gelatine, Glycerol, Polyethylenglycol, Hartfett, Cetostearylalkoholpolyethylenglycolether, Natriumdodecylsulfat, Glycerol(mono,di,tri)fettsäureester(C12-C18)-Polyethylenglycoldodecylether Gemisch, Paraffin, Ethyl-4-hydroxybenzoat, Propyl-4-hydroxybenzoat und Vaseline.

Die Vaginaltabletten können in Form von Einschicht-, Zweischicht- oder Dreischichttabletten oder als Tabletten mit Brausesatz vorliegen.

Die neue Kombination kann zur Behandlung einer Vielzahl von vaginalen Erkrankungen eingesetzt werden. So können die am häufigsten auftretende Kolpitiden, die Candida-Kolpitis (Pilzinfektion) und die bakterielle Vaginose ( bakterielle Mischinfektion) erfolgreich behandelt werden.

Des weiteren erlaubt die erfindungsgemäße Kombination die Behandlung sogenannter Mischinfektionen, die durch Bakterien und Pilze hervorgerufen werden. Bei der Applikation der erfindungsgemäßen Kombination werden gleichzeitig beide Krankheitserreger bekämpft.

Unklare Infektionen erfordern üblicherweise äußerst aufwendige diagnostische Bestimmungen. Bei der Behandlung solcher Erkrankungen mit der Kombination aus Clindamycin und Clotrimazol reduziert sich dieser Aufwand erheblich, da es praktisch unerheblich ist, welche Art der Kolpitis vorliegt.

Bei der Monotherapie einer Infektion mit einem Antibiotikum nach dem Stand der Technik ist die am häufigsten auftretende Sekundärinfektion eine Candidose. Mit dem Einsatz der erfindungsgemäßen Kombination bei der Behandlung lassen sich solche Sekundärinfektionen verhindern. Dadurch erübrigt sich eine weitere Behandlung, die zum einen für den Patienten sehr unerfreulich und mit einer verlängerten Behandlungsdauer verbunden ist und zum anderen kann durch die Kombinationstherapie vom gesundheitspolitischen Standpunkt der finanzielle Aufwand deutlich verringert werden.

Die Erfindung wird durch nachstehende Beispiele näher erläutert, ohne aber den Erfindungsumfang damit einzuschränken.

### Beispiel 1:

| Einschichttablette | |
|---|---|
| Clotrimazol | 100,0 mg |
| Clindamycin-HCL (entspricht 20 mg Clindamycin) | 22,7 mg |
| Lactose D20 | 265,0 mg |
| Maisstärke | 33,3 mg |
| Hydroxypropylcellulose (HPC) (Klucel EF) | 2,0 mg |
| Calciumlactat x 5 H₂O | 30,0 mg |
| Milchsäure | 41,0 mg |
| Mikrokristalline Cellulose (Avicel PH 102) | 128,0 mg |
| Kollidon CL | 12,0 mg |
| Aerosil 200 | 7,0 mg |
| Magnesiumstearat | 7,0 mg |

Clotrimazol, Clindamycin-HCl, Lactose, ein Teil der Maisstärke, HPC, Calciumlactat und Milchsäure werden in einem Wirbelschichtgranulator granuliert. Das erhaltene Granulat sowie der restliche Teil der Maisstärke, Kollidon, mikrokristalline Cellulose, Magnesiumstearat und Aerosil werden durch ein Zwangssieb (1,25 mm) gegeben und in einem Containermischer homogenisiert. Die erhaltene Mischung wird auf einer Rundlauftablettenmaschine zu Tabletten verpreßt.

### Beispiel 2:

| Einschichttablette : | |
|---|---|
| Clotrimazol | 100,0 mg |
| Clindamycin-HCL (entspricht 20 mg Clindamycin) | 22,7 mg |
| Lactose D20 | 265,0 mg |
| Maisstärke | 33,3 mg |
| HPC (Klucel EF) | 2,0 mg |
| Calciumlactat x 5 H₂O | 90,0 mg |
| Milchsäure | 35,0 mg |
| Mikrokristalline Cellulose (Avicel PH 102) | 128,0 mg |
| Kollidon CL | 12,0 mg |
| Aerosil 200 | 7,0 mg |
| Magnesiumstearat | 7,0 mg |

Mit den vorstehend angegebenen Bestandteilen wird eine Tablette in Analogie zu Beispiel 1 hergestellt.

### Beispiel 3:

| Einschichttablette: | |
|---|---|
| Clotrimazol | 100,0 mg |
| Clindamycin-HCL (entspricht 20 mg Clindamycin) | 22,7 mg |
| Lactose D20 | 795,3 mg |
| Maisstärke | 100,0 mg |
| HPC (Klucel EF) | 5,0 mg |
| Calciumlactat x 5 H₂O | 30,0 mg |
| Milchsäure | 70,0 mg |
| Mikrokristalline Cellulose (Avicel PH 102) | 384,5 mg |
| Kollidon CL | 35,0 mg |
| Aerosil 200 | 23,0 mg |
| Magnesiumstearat | 22,5 mg |

Clotrimazol, Clindamycin-HCl, Lactose, ein Teil der Maisstärke, ein Teil der mikrokristallinen Cellulose, HPC, Calciumlactat und Milchsäure werden in einem Wirbelschichtgranulator granuliert. Das erhaltene Granulat sowie der restliche Teil der Maisstärke und der mikrokristallinen Cellulose, Kollidon, Magnesiumstearat und Aerosil werden durch ein Zwangssieb (1,25 mm) gegeben und in einem Containermischer homogenisiert. Die erhaltene Mischung wird auf einer Rundlauftablettenmaschine zu Tabletten verpreßt.

### Beispiel 4:

| Zweischichttablette: | | |
|---|---|---|
| | *1. Schicht* | *2. Schicht* |
| Clotrimazol | 100,0 mg | ------------ |
| Clindamycin-HCL (entspricht 20 mg Clindamycin) | ------------ | 22,7 mg |
| Lactose D20 | 265,0 mg | 265,0 mg |
| Maisstärke | 33,0 mg | 33,6 mg |
| HPC (Klucel EF) | 2,0 mg | 2,0 mg |
| Calciumlactat x 5 H₂O | 90,0 mg | 10,0 mg |
| Milchsäure | 35,0 mg | 23,0 mg |
| Mikrokristalline Cellulose (Avicel PH 102) | 128.0 mg | 128,0 mg |
| Kollidon CL | 12.0 mg | 12,0 mg |
| Aerosil 200 | 7.0 mg | 7,7 mg |
| Magnesiumstearat | 7,0 mg | 7,0 mg |

Mit den oben genannten Bestandteilen wird zum einen ein Granulat für die erste Schicht und zum anderen ein Granulat für die zweite Schicht analog Beispiel 1 hergestellt und zu einer Zweischichttablette verpreßt.

### Beispiel 5:

| Zweischichttablette: | | |
|---|---|---|
| | *1. Schicht* | *2. Schicht* |
| Clotrimazol | 100,0 mg | ------------ |
| Clindamycin-HCL (entspricht 20 mg Clindamycin) | ------------ | 22,7 mg |
| Lactose D20 | 265,0 mg | 265,0 mg |
| Maisstärke | 33,0 mg | 33,6 mg |
| HPC (Klucel EF) | 2,0 mg | 2,0 mg |
| Calciumlactat x 5 H₂O | 90,0 mg | ------------ |
| Milchsäure | 35,0 mg | ------------ |
| Mikrokristalline Cellulose (Avicel PH 102) | 128,0 mg | 128,0 mg |
| Kollidon CL | 12,0 mg | 12,0 mg |
| Aerosil 200 | 7,0 mg | 7,7 mg |
| Magnesiumstearat | 7,0 mg | 7,0 mg |

Mit den vorstehend angegebenen Bestandteilen wird eine Tablette in Analogie zu Beispiel 4 hergestellt.

### Beispiel 6:

| Einschichttablette: | |
|---|---|
| Clotrimazol | 100,0 mg |
| Clindamycin-HCL (entspricht 10 mg Clindamycin) | 11,4 mg |
| Lactose D20 | 265,0 mg |
| Maisstärke | 33,6 mg |
| HPC (Klucel EF) | 2,0 mg |
| Calciumlactat x 5 H₂O | 90,0 mg |
| Milchsäure | 35,0 mg |
| Mikrokristalline Cellulose (Avicel PH 102) | 128,0 mg |
| Kollidon CL | 12,0 mg |
| Aerosil 200 | 7,0 mg |

Mit den vorstehend angegebenen Bestandteilen wird eine Tablette in Analogie zu Beispiel 1 hergestellt.

### Beispiel 7:

| Dreischichttablette: | | |
|---|---|---|
| | *1. Schicht* | *3. Schicht* |
| Clotrimazol | 100,0 mg | ------------ |
| Clindamycin-HCL (entspricht 20 mg Clindamycin) | ------------ | 22,7 mg |
| Lactose D20 | 265,0 mg | 265,0 mg |
| Maisstärke | 33,0 mg | 33,6 mg |
| HPC (Klucel EF) | 2,0 mg | 2,0 mg |
| Calciumlactat x 5 H₂O | 90,0 mg | ------------ |
| Milchsäure | 35,0 mg | ------------ |
| Mikrokristalline Cellulose (Avicel PH 102) | 128,0 mg | 128,0 mg |
| Kollidon CL | 12,0 mg | 12,0 mg |
| Aerosil 200 | 7,0 mg | 7,7 mg |
| Magnesiumstearat | 7,0 mg | 7,0 mg |

Die zweite Schicht stellt eine Zwischenschicht dar, die sich zwischen der ersten und dritten Schicht befindet.

| Zwischenschicht: | |
|---|---|
| Lactose (Tablettose) | 58,7 mg |
| Avicel PH 102 | 30,0 mg |
| Aerosil | 0.5 mg |
| Magnesiumstearat | 0,8 mg |
| Maisstärke | 10,0 mg |

### Beispiel 8:

| Zweischichttablette: | | |
|---|---|---|
| | *1. Schicht* | *2. Schicht* |
| Clotrimazol | 100,0 mg | ------------ |
| Clindamycin-HCL (entspricht 20 mg Clindamycin) | ------------ | 22,7 mg |
| Lactose D20 | 265,0 mg | 93,7 mg |
| Maisstärke | 33,0 mg | 20,0 mg |
| HPC (Klucel EF) | 2,0 mg | ------------ |
| Calciumlactat x 5 H₂O | 90,0 mg | ------------ |
| Milchsäure | 35,0 mg | ------------ |
| Mikrokristalline Cellulose (Avicel PH 102) | 128,0 mg | 60,0 mg |
| Kollidon CL | 12,0 mg | ------------ |
| Aerosil 200 | 7,0 mg | 1,6 mg |
| Magnesiumstearat | 7,0 mg | 2,0 mg |

Mit den vorstehend angegebenen Bestandteilen wird die erste Schicht in Analogie zu Beispiel 1 granuliert. Die clindamycinhaltige Schicht wird nicht granuliert, sondern direkt mit dem Granulat der ersten Schicht zu einer Zweischichttablette verpreßt.

### Beispiel 9:

| Tablette mit Brausesatz (Einschicht): | |
|---|---|
| Clotrimazol | 100,0 mg |
| Clindamycin-HCL (entspricht 20 mg Clindamycin) | 22,7 mg |
| Lactose D20 | 265,0 mg |
| Maisstärke | 33,3 mg |
| HPC (Klucel EF) | 2,0 mg |
| Calciumlactat x 5 H₂O | 90,0 mg |
| Milchsäure | 35,0 mg |
| Mikrokristalline Cellulose (Avicel PH 102) | 120,0 mg |
| Citronensäure | 50,0 mg |
| Adipinsäure | 10,0 mg |
| Aerosil 200 | 7,0 mg |
| Magnesiumstearat | 7,0 mg |
| Natriumhydrogencarbonat | 30,0 mg |

Clotrimazol, Clindamycin-HCl, Lactose, Maisstärke, HPC, Calciumlactat und Milchsäure werden in einem Wirbelschichtgranulator granuliert.
Das erhaltene Granulat wird mit den übrigen Brause- und/ oder Zerfallskomponenten sowie mit mikrokristalliner Cellulose, Magnesiumstearat und Aerosil gemischt und zu Tabletten verpreßt.

### Beispiel 10:

| Vaginalcreme: | |
|---|---|
| Eine Appliziereinheit beträgt 5 Gramm. Diese enthält 100 mg Clotrimazol und 20 mg Clindamycin. | |

| *Ein Gramm Creme ist entsprechend zusammengesetzt:* | |
|---|---|
| Clotrimazol | 20,0 mg |
| Clindamycin-HCl (entspricht 4 mg Clindamycin) | 4,54 mg |
| Sorbitanmonostearat | 20,0 mg |
| Polysorbat 60 (Tween 60) | 15,0 mg |
| Cetylpalmitat (Cutina CP-A) | 30,0 mg |
| Dickflüssiges Paraffin | 130,46 mg |
| Cetylstearylalkohol | 100,0 mg |
| Benzylalkohol | 10,0 mg |
| Gereinigtes Wasser | 670,0 mg |

### Beispiel 11:

| Ovulum (100/20): | |
|---|---|
| Pro Ovulum werden folgende Bestandteile zusammengefügt: | |
| Clotrimazol | 100,0 mg |
| Clindamycin- HCl (entspricht 20 mg Clindamycin) | 22,7 mg |
| Calciumlactat x 5H₂O | 77,3 mg |
| Gelantine | 250,0 mg |
| Gereinigtes Wasser | 250,0 mg |
| Glycerol | 1250,0 mg |

### Beispiel 12:

| Ovulum (100/20): | |
|---|---|
| Pro Ovulum werden folgende Bestandteile zusammengefügt: | |
| Clotrimazol | 100,0 mg |
| Clindamycin- HCl (entspricht 20 mg Clindamycin) | 22,7 mg |
| Calciumlactat x 5H₂O | 77,3 mg |
| Macrogol 400 | 1000,0 mg |
| Macrogol 6000 | 800,0 mg |
| Milchsäure | 200,0 mg |

### Beispiel 13:

| Ovulum/ Suppositorium | |
|---|---|
| Pro Ovulum werden folgende Bestandteile zusammengefügt: | |
| Clotrimazol | 100,0 mg |
| Clindamycin- HCl (entspricht 20 mg Clindamycin) | 22,7 mg |
| Calciumlactat x 5H₂O | 77,3 mg |
| Hartfett | 1800,0 mg |

### Beispiel 14:

| Ovulum (100/20): | |
|---|---|
| Pro Ovulum werden folgende Bestandteile zusammengefügt: | |
| Clotrimazol | 100,0 mg |
| Clindamycin- HCl (entspricht 20 mg Clindamycin) | 22,7 mg |
| Calciumlactat x 5H₂O | 77,3 mg |
| Hartfett | 1780,0 mg |
| Cetomacrogol 1000 | 20,0 mg |

### Beispiel 15:

Die erfindungsgemäße pharmazeutische Zusammensetzung wurde in einer Studie bei 25 Patientinnen angewandt. Bei diesen 25 Patientinnen wurde in 20 Fällen eine bakterielle Mischinfektion, in 3 Fällen eine Mischinfektion, hervorgerufen durch Bakterien und Pilze und in 2 Fällen eine reine Pilzinfektion festgestellt.

Nach einer 6-tägigen Therapie frisch zubereiteten Vaginalovula, die 20 mg Clindamycin und 100 mg Clotrimazol enthielten, wurde bei 23 Patientinnen eine vollständige Ausheilung der Infektion erzielt. Es konnten des weiteren keine Sekundärinfektionen festgestellt werden.

## Patentansprüche

1. Pharmazeutische Zusammensetzung zur vaginalen Verabreichung enthaltend 10-20 mg Clindamycin und 50-100 mg Clotrimazol pro Applikationseinheit sowie mindestens einen pharmazeutischen Träger.

2. Pharmazeutische Zusammensetzung nach Anspruch 1 zur Behandlung vaginaler Infektionen.

3. Pharmazeutische Zusammensetzung nach Anspruch 1 oder 2 in Form einer Einschichttablette, Brausetablette, Zweischichttablette, Dreischichttablette, Vaginalring, Suppositorium oder Ovulum.

4. Pharmazeutische Zusammensetzung nach Ansprüchen 1-3 in Form einer halbfesten Formulierung.

5. Verfahren zur Herstellung einer pharmazeutischen Zusammensetzung nach einem der Ansprüche 1-4, bei dem man die aktiven Bestandteile Clindamycin und Clotrimazol mit mindestens einem pharmazeutischen Träger vereinigt.

## Claims

1. A pharmaceutical composition for the vaginal administration comprising 10-20 mg of clindamycine and 50-100 mg of clotrimazole per administration unit and at least one pharmaceutical carrier.

2. A pharmaceutical composition according to claim 1 for the treatment of vaginal infections.

3. A pharmaceutical composition according to claim 1 or 2 in the form of a single-layer tablet, an effervescent tablet, a two-layer tablet, a three-layer tablet, a vaginal ring, a suppository or an ovulum.

4. A pharmaceutical composition according to claims 1-3 in the form of a semisolid formulation.

5. A method for the preparation of a pharmaceutical composition according to one of the claims 1-4, wherein the active components clindamycine and clotrimazole are combined with at least one pharmaceutical carrier.

## Revendications

1. Composition pharmaceutique destinée à l'application vaginale, contenant 10-20 mg de clindamycine et 50-100 mg de clotrimazole par unité d'application ainsi qu'au moins un support pharmaceutique admissible.

2. Composition pharmaceutique selon la revendication 1 pour le traitement d'infections vaginales.

3. Composition pharmaceutique selon les revendications 1 ou 2, sous forme d'un comprimé monocouche, effervescent, bicouche, tricouche, pessaire vaginal, suppositoire ou ovule vaginaux.

4. Composition pharmaceutique selon les revendications 1 - 3 présentée sous forme d'une formulation semi-solide.

5. Procédé pour la fabrication d'une composition pharmaceutique selon une des revendications 1-4, au cours duquel on associe les substances actives de la clindamycine et du clotrimazole avec au minimum un support pharmaceutique.
